# EUROPEAN PATENT SPECIFICATION

(11) **EP 2 083 689 B1**
(45) Date of publication and mention of the grant of the patent: **04.08.2010**
(21) Application number: 07760065.8
(22) Date of filing: 04.04.2007
(51) Int. Cl.: A61B 5/151

(54) **DIAGNOSTIC TEST ELEMENT AND PROCESS FOR ITS PRODUCTION**
DIAGNOSTISCHES TESTELEMENT UND VERFAHREN ZU SEINER HERSTELLUNG
ELEMENT DE TEST DIAGNOSTIQUE ET PROCEDE DE PRODUCTION

(30) Priority: 15.10.2006 WO PCT/EP2006/009945
(43) Date of publication of application: 05.08.2009
(73) Proprietor: Roche Diagnostics GmbH, 68305 Mannheim (DE); F. Hoffmann-La Roche AG, 4070 Basel (CH)
(72) Inventor: HAAR, Hans-Peter, 67105 Wiesloch (DE); SCHWIND, Karin, 67105 Schifferstadt (DE); MARQUANT, Michael, 68309 Mannheim (DE); SURRIDGE, Nigel, Carmel, IN 46032 (US)
(74) Representative: Pfiz, Thomas
(86) International application number: PCT/US2007/065918
(87) International publication number: WO 2008/048709

(56) References cited:
- EP-A- 0 073 558
- EP-A- 1 491 143
- US-A- 6 032 059
- US-B1- 6 375 627

## Description

### Technical Field

The invention generally concerns a process for producing a diagnostic test element suitable for analyzing a body fluid. The diagnostic test element has a lancing member that can puncture a body part to obtain a body fluid sample. The lancing member is provided with a capillary collecting channel for body fluid obtained by the puncture. The test element also has a sensor member for an optical or electrochemical measurement is connected to the lancing member.

### Background

Blood sugar self-monitoring is usually carried out several times daily as part of an insulin treatment regimen to control diabetes. It is therefore desirable to minimize the number of handling steps the patient is required to carry out and to ensure a relatively painless and highly reliable blood sugar measurement. Disposable measurement articles are used for hygienic reasons. In conventional blood sugar measurements, samples are generated by finger pricking with lancets and the measurement is carried out on separate detection elements. This requires a large number of handling steps by the patient which can result in errors. Further disadvantages are blood volumes that are too large, non-robust sample transfer procedures, and lack of integration that requires the patient to organize and handle separate devices and disposable supplies.

### Summary

Taking this as a starting point, the object of the invention is to further improve the test elements and processes for their production known in the prior art, and in particular to enable practicable mass production and use of more highly integrated test elements that are cost-effective and at the same time reliable; while also allowing, relatively simple instrument technology.

The combination of features stated in the independent claims is proposed to achieve this object. Advantageous embodiments and further developments of the invention are derived from the dependent claims.

The idea behind the invention is to use test elements in which a small volume sample can be collected and reliably analyzed. A production process is proposed for such test elements in which a lancing member that can puncture a body part is provided with a collecting channel, preferably exhibiting capillary action, for the body fluid obtained by the puncture, and a sensor member for an optical or electrochemical measurement is connected to the lancing member. According to the invention the sensor member and the lancing member are joined together as interlocking connecting components, such that a measuring element of the sensor member is inserted into the collecting channel through an insertion opening in the lancing member. This permits the parts to be separately produced in optimized manufacturing steps at low production costs and subsequently integrated into a compact configuration by a simple interlocking connection in this configuration a small amount of sample in the collecting channel is sufficient to reliably contact and wet the sensor located therein. This also allows precise positioning tolerances in the test element production process, thereby simplifying tolerance management on the instrument side. In particular, it is not necessary to actively transfer the sample to a separate sensor that is not in the direct sample flow, thereby making possible reliable measurements, even with very small samples of 100 nanolitres or less. Reliable measurement is of special importance because automation of handling steps by the system reduces the demand of ability of the patient to monitor and control the process.

The invention is described in more detail in the following with reference to the embodiment examples shown schematically in the drawing.

### Brief Description of the Drawings

- Fig. 1: shows a blood sugar measuring instrument with a diagnostic test element used therein as a consumable unit in a diagrammatic representation;
- Fig. 2: shows an embodiment of the test elements in a partial perspective representation;
- Fig. 3: shows an exploded diagram of the test element according to fig. 2 comprising a lancing member, a sensor based on wire electrodes and a holder,
- Fig. 4: shows an embodiment of an optical test element in a perspective view;
- Figs. 5 and 6: show various production steps for the test element according to fig. 4;
- Figs. 7 and 8: show another embodiment of an electrochemical test element in a representation corresponding to figs. 2 and 3;
- Fig. 9: shows the sensor member of the test element according to fig. 7 in a partial side-view;
- Figs. 10 to 12: show another embodiment of an electrochemical test element in a representation corresponding to figs. 7 to 9;
- Fig. 13: shows a device for the continuous manufacture of a light guide composite member of the test element;
- Fig. 14: shows an enlarged section of fig. 13;
- Fig. 15: shows the light guide composite member in a perspective view;
- Fig. 16: shows a device for producing label-like measuring elements for the test element in a side-view;
- Figs. 17 and 18: show enlarged sections of fig. 16;
- Fig. 19: shows a device for equipping test elements with lancing members in a diagrammatic representation;
- Fig. 20: shows a sectional top-view of the device according to fig. 19; and
- Fig. 21: shows a device for hydrophilically coating the lancing members of the test elements.

### Detailed Description

The test elements 10 shown in the drawing can be used as consumables for blood sugar measurement in a hand-held device 12 designed for this purpose wherein glucose can be detected directly in the test element using a minimal amount of sample. For this purpose the test elements 10 comprise a lancing member 14 having a slot-shaped collecting channel 16. a sensor member 18 for an optical or electrochemical measurement in the collecting- channel 16, and a holder 20 for the lacing member and the sensor member.

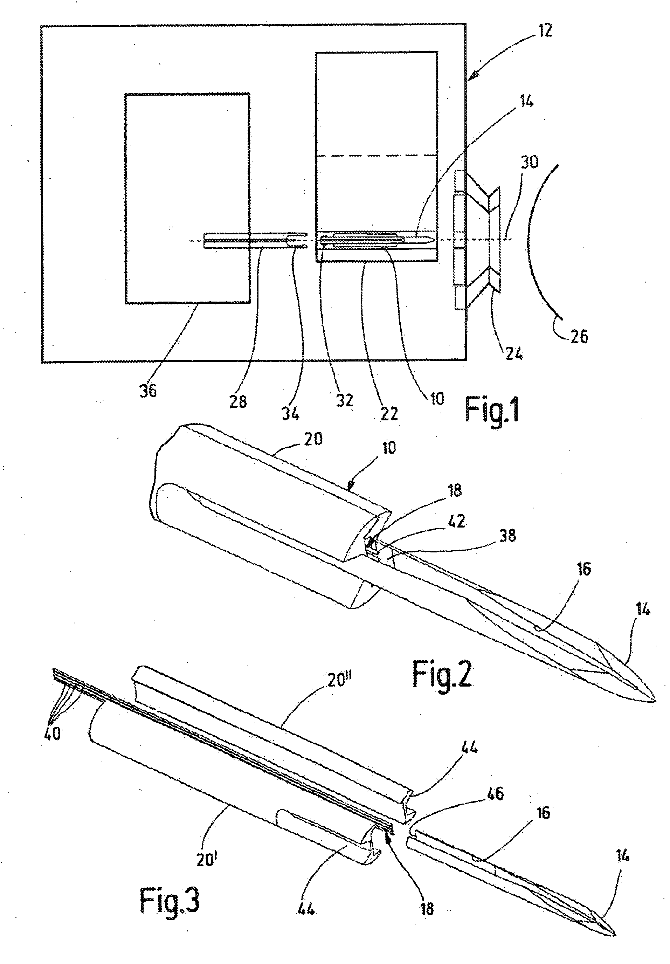

As illustrated in fig. 1 a plurality of test elements 10 in, for example, a drum-like magazine 22 can be moved successively into an active working position with respect to a ring-shaped support 24 with a puncture opening for a finger tip 26 of a user. A lancing drive 28 which engages into the magazine 22 enables a reciprocating lancing movement of the test element 10 along a lancing axis 30. In this connection the tip of the lancing member 14 points in the distal direction towards the body part while a coupling end 32 of the test element is coupled to a suitable gripper member 34 of the lancing drive 28 for mechanical drive and signal coupling, The body fluid (blood or tissue fluid) which is taken up in the collecting channel 16 by the lacing process, can be directly optically or electrochemically analyzed by the sensor member 18. The signal analysis takes place in an evaluation unit36 in the instrument. This also enables the result of the measurement to be displayed to the user so that the blood sugar can be checked on the spot without complicated handling steps.

As shown in figs. 2 and 3 the shaft-like elongate lancing member 14 has a transverse continuous longitudinal slot forming the collecting channel 16. This enables, optionally by means of capillary action, the transfer of a microscopic amount of liquid into the proximal measuring zone 38. The elongate slit opening on both sides ensures an effective liquid uptake without the risk of blockage by cell components. In order to collect blood as gently and pain-free as possible, the collecting channel 16 is designed to have a volume of only a few tens of nanolitres.

According to fig. 3 the sensor member 18 has several parallel electrode wires 40 in order to achieve a high degree of measurement reliability through redundant detection of measured values. The electrode wires are ether continuously coated with a test reagent, or only coated at the ends in order to enable electrochemical detection of the analyte in the body fluid. For this purpose the coated ends of the wire protrude in a self-supporting manner into the internal cross-section of the collecting channel 16 in the area of the measuring zone 38. In this manner the wire ends form a measuring element 42 which is electrically insulated inside the channel from the channel wall by a free space, and body fluid can flow against and wet the front of the measuring element. In this arrangement a few nanolitres of blood are sufficient for an integrated detection of measured values in the test element 10.

Some specific methods for manufacturing test elements 10 configured in this manner are described in the following. In general the sensor member 18 and the lancing member 14 are joined in an interlocking manner whereby the measuring element 42 is inserted into the collecting channel 16.

In the embodiment according to figs. 2 and 3 the electrode wires 40 are clamped between two halves 20', 20" of the longitudinally divided holder 20 whereby grooves that are not shown ensure well-defined positioning of the wires. Then the fork-shaped end of the lancing member 14 which is prefabricated from a metallic wire material by a combination of grinding and laser cutting processes or etching is mounted axially on the receiving grooves 44 of the holder 20. In this process the measuring element 42 of the sensor member 18 is inserted via the proximal slot opening 46 into the collecting channel 16 and moved into the specified position.

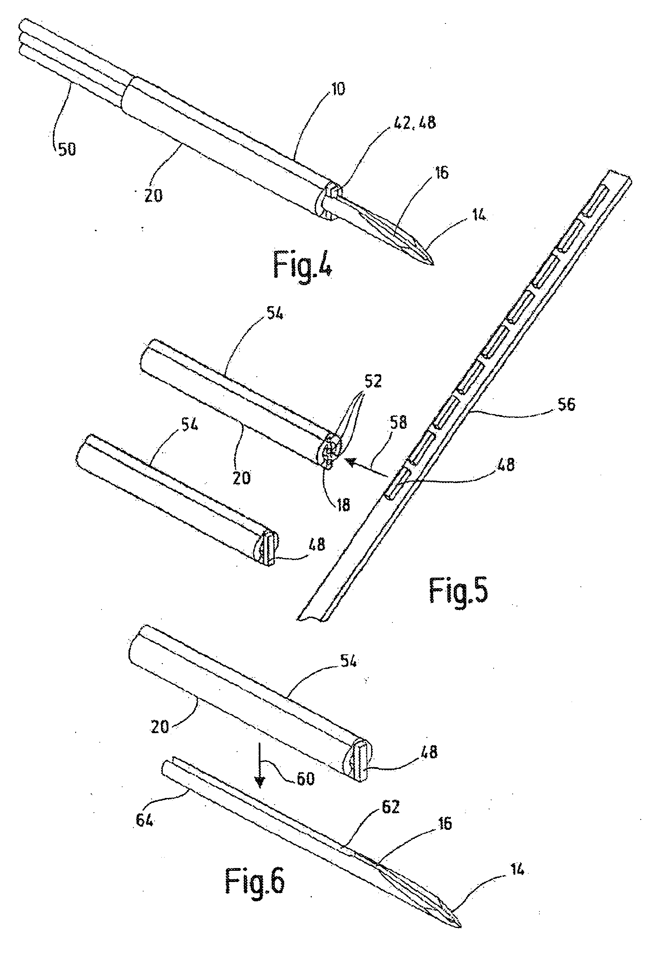

Fig. 4 shows another variant of a test element 10 which is equipped with optical light guides and a reagent pad 48 as a measuring element 42 for a reflection-photometric measurement. The signal is coupled out by an endface contact with light guides 50 on the instrument side which have a suitable diameter for a reliable signal transmission.

As shown in fig. 5 a composite element 54 consisting of three parallel light guides 52 and a plastic holder 20 is manufactured by coextrusion and then subsequent division into sections. The distal front end of the composite part 54 prefabricated in this manner is then provided with a reagent pad 48 which is dispensed from a tape 56 like a self-adhesive label and glued onto the free ends of the light guides (arrow 58).

In another manufacturing step illustrated in fig. 6 the composite part 54 is subsequently clipped or latched onto the lancing member 14 in the direction of the arrow 60. In this process the reagent pad 48 is also inserted transversely into the collecting channel 16 via the longitudinal slit opening 62 to obtain the configuration shown in fig. 4. The holder 20 embraces the shaft 64 of the lancing member 14 which is slotted and elongated on the rear side, in a shell-shaped manner. This facilitates low-cost production because holder 20 can be combined with the lancing member 14 by a fast snap-on process. When used for measurement the central fiber of the three fibers comprising light guide 52 enables irradiation of the measuring light which is reflected from the rear side of the reagent pad 48 and is detected via the two outer fibers of light guide 52 for a duplicate photometric measurement.

It is also conceivable that the light guides or the reagent pad 48 are provided with a fluorescent indicator as described in the patent application WO 03/097859 and to which explicit reference is herewith made. Specifically, a liquid polymerizable composition comprising a detection reagent can be applied. After application of the sample to the front side of such a sensor, exciting light, e.g. UV light, is beamed in through a light guide. The fluorescence, e.g. bluelight, generated through the reaction of the analyte with the detection reagent in the polymer layer is detected via the light guide with a detector. Preferably, the polymer layer has a thickness of about 50 microns or less, which allows for comparably short reaction times for generating the fluorescence light when the analyte is detected. In this way, the reaction or measurement time can be shorter than 2s, preferably shorter than 1s, thereby enabling a measurement while the lancing member is still in the skin of the body part. It has been found that leaving the lancing member inserted for such a short time Interval is fully acceptable for most users.

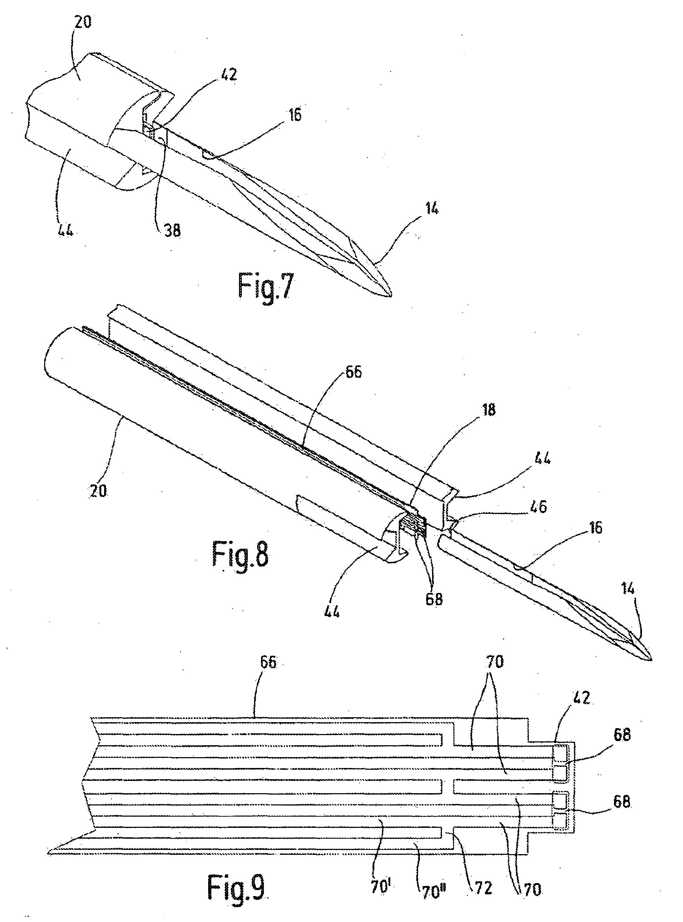

The embodiment shown in figs. 7 to 9 differs from the example of figs. 2 and 3 essentially in that instead of coated electrode wires, a printed circuit board 66 is used as a prefabricated sensor member 18. The distal end of the printed circuit board 66 is provided with two electrically connected reagent fields 68 which protrude freely into the measuring zone 38 to form a measuring element 42, Also in this case it is manufactured by first clamping the printed circuit board 66 into the two-part holder 20 and then axially mounting the lancing member 1-4 so that the fork-shaped ends of the lancing member 14 engage in the grooves 44 and the measuring element 42 is positioned in the channel cross-section.

Fig. 9 shows the electrical connection of the reagent fields 68 via in this case two conductor paths 70 on the printed circuit board 66. Each of the conductor paths 70 branch over one conductor bridge 72 into a primary conductor path 70' and a secondary conductor path 70" in order to thus be able to carry out an electrical continuity test at least up to the conductor bridge 72 and to detect any interruptions of the conductor paths. This, in addition to the redundant double measurement, further increases the functional reliability.

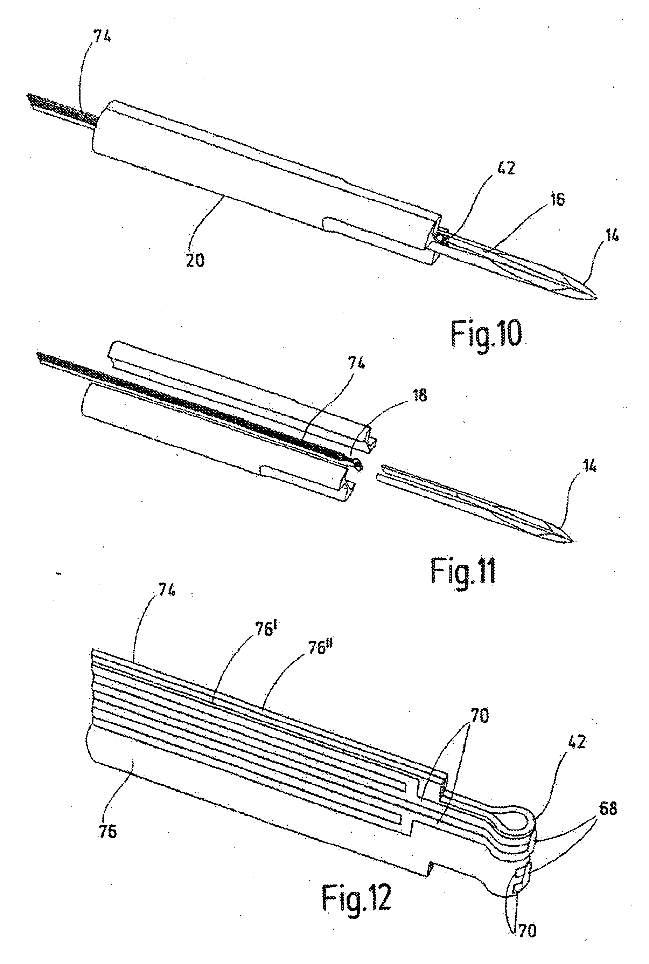

A similar embodiment to figs. 7 to 9 is shown in figs. 10 to 12. However, instead of the printed circuit board 66, a test strip 74 which is folded transversely is provided as a sensor element 18. It is again assembled as described above by clamping the test strip 74 into the holder 20 and axially mounting the lancing member 14.

As also illustrated in fig. 12, the concept of the folded test strip 74 has various advantages. The manufacture can start with a planar structure in the form of a thin flexible foil strip. This is provided with conductor paths 70 and with reagent fields 68 at their contact ends while still in an unfolded form. Subsequently the equipped foil strips are folded up in the middle so that the strip halves 76', 76" can be glued together. The reagent fields 68 then protrude freely from the front face in the area of the rounded bending site so that liquid can flow against and wet the reagent fields in the channel 16.

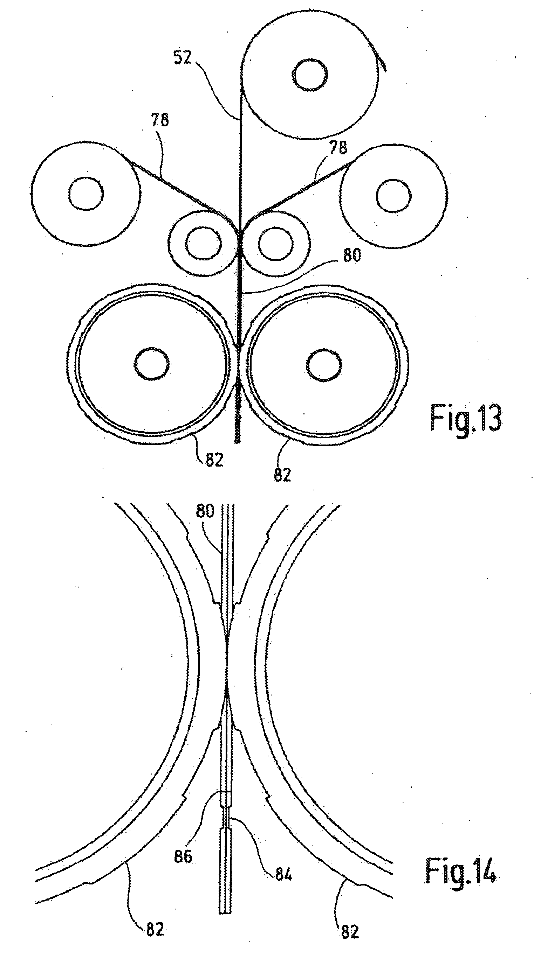

Special process steps are illustrated in figs. 13 to 21 which allow an advantageous continuous mass production through different stations.

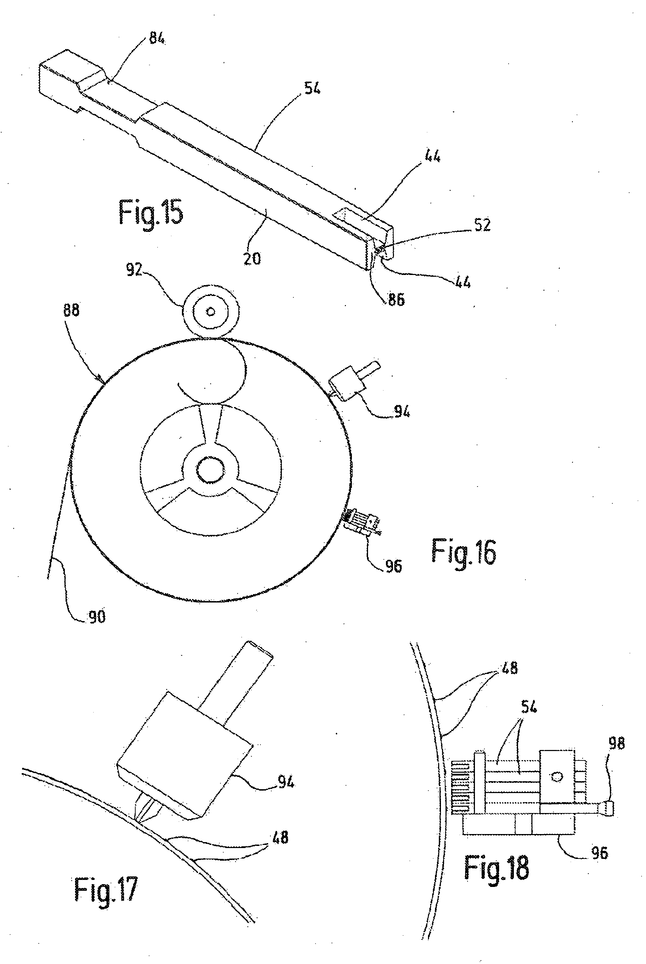

Firstly according to fig. 13 three parallel light guides 52 in the form of polymer fibers are fed from reels and laminate between the foil strips 78 to form a composite strand 80. In a subsequent embossing station the outer contour of the composite strand 80 is shaped by embossing rollers 82. As shown in fig. 14 this enables both the receiving grooves 44 and a coupling structure 84 for a positive locking gripper coupling to be created. The reshaped composite strand 80 is subsequently severed at the cutting lines 86 to thus obtain the composite parts 54 shown in fig. 15.

The distal front end 86 of the composite part 54 is equipped with measuring elements in the rotary station 88 shown in fig. 16. The reagent pads 84 provided for this should have an adequate homogeneity in the required small dimensions - for example 200 x 400 µm For this purpose a test chemistry is firstly knife-coated over a large area of a wide test tape 90. Then the test tape 90 is reduced to a narrow central strip by parallel cutting rollers 92. As shown in fig. 17, the desired reagent pads 48 can be cut out of the central strip by a punching device 94. The reagent pads 48 are finally applied to the composite parts 54 at another rotary position of the rotary station 88. For this purpose a transfer device 96 is provided in which a plurality of composite parts 54 are stacked. The transfer takes place by means of a pneumatic unit 98 which successively brings the composite parts 54 into an end face contact with the self-adhesive reagent pads 48 by means of a blast of air.

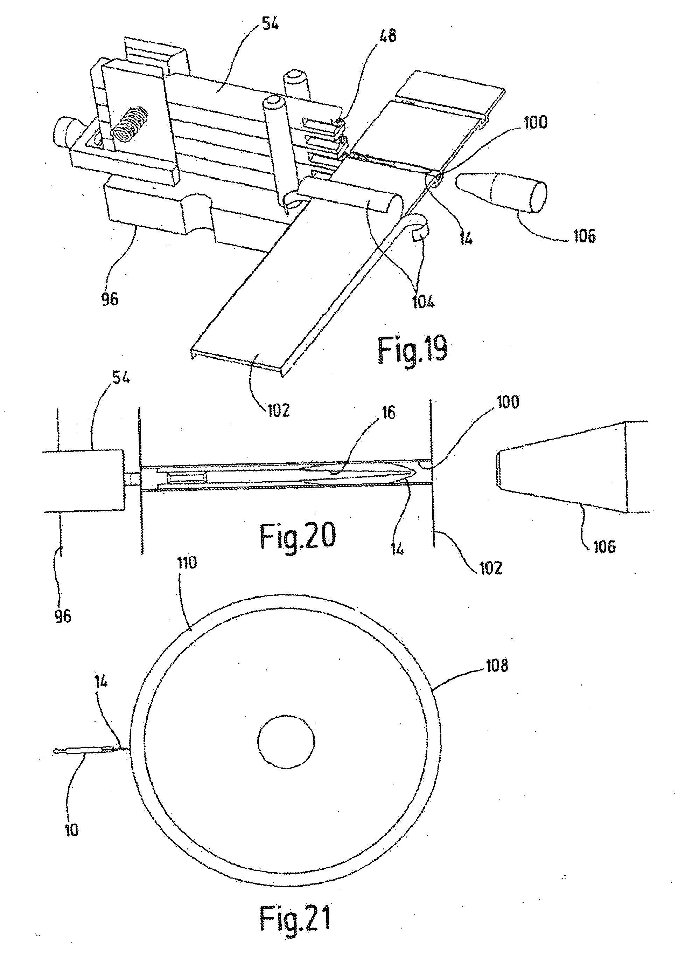

The composite parts 54 prepared in this manner are provided with lancing members 14 in the assembly station shown in figs. 19 and 20. The prefabricated lancing members 14 can in this process be conveyed to the transfer device 96 in depressions 100 of a blister tape 102. The test element storage in the depressions 100 in a blister tape 102 sealed by cover foils 104 also enables a separate sterilization of the lancing members 14. for example by irradiation, without damaging the test chemistry. The axial sliding assembly motion of the lancing members 14 onto the composite parts 54 is in turn effected by means of a blast of air by a pneumatic unit 106.

The metallic lancing members 14 can be provided with a hydrophilic layer in order to support the uptake of body fluid in the collecting channel 16. For this purpose the lancing members 14, either before or after they are mounted on the finally packaged test elements 10, can be brought into contact with an absorbent application ring 110 at the application station 108 shown in fig. 21, the ring being impregnated with a suitable hydrophilic coating substance. The amount of substance applied to the lancing member 14 can then be accurately controller by means of the contact time.

A further possibility to produce the light fiber structure is to generate the fibers in situ on or within an embossed part of a tape or foil-like carrier. In this process a thin layer of low refractive index transparent polymer, for example epoxy, is deposited onto a structural substrate to form a base layer. This is then over-coated by a thin layer of photosensitive high refractive index transparent polymer. This layer is processed by UV photolithography to selectively remove material, leaving the light guides as generally parallel strips of high refractive index polymer bonded to the low refractive index base layer. Finally, a layer of low refractive index polymer is flow-coated over the light guides and polymerized to form a solid layer. The result is that high refractive index light guides are surrounded on all sides by lower refractive index transparent polymer, forming functional independent light guides. Suitable materials are available commercially, for example, under the tradenames EpoCore and EpoOad epoxy polymer resins supplied by the German company Micro Resist Technology. Particular advantages of such photolithographic processes for manufacturing the parallel light guides include low cost volume production and the ability to vary the geometry of the light guides along their length. For example, the light guides may be tapered to transition from a small reagent pad to a larger optical interface with the measuring instrument.

## Claims

1. Process for producing a diagnostic test element (10) for analyzing a body fluid in which a lancing member (14) that can puncture a body part is provided with a preferably capillary collecting channel (16) for body fluid obtained by the puncture and a sensor member (18) for an optical or electrochemical measurement is connected to the lancing member, **characterized in that** the sensor member (18) and the lancing member (14) can be joined together as interlocking connecting components wherein a measuring element (42) of the sensor member is inserted into the collecting channel through an insertion opening (46) of the lancing member.

2. Process according to claim 1, **characterized in that** the sensor member and the lancing member can be joined together along a connecting axis running in the longitudinal direction of the collecting channel.

3. Process according to claim 1 or 2, **characterized in that** the collecting channel is formed as a preferably transverse continuous slot in the lancing member, said slot being open at a proximal aperture, and that the sensor member is inserted into the slot through the proximal aperture.

4. Process according to claim 1, **characterized in that** the sensor member is inserted transversely into the collecting channel which has an open longitudinal side.

5. Process according to one of the claims 1 to 4, **characterized in that** the measuring element in the interior of the collecting channel is arranged such that it is galvanically separated from the channel wall.

6. Process according to one of the claims 1 to 5, **characterized in that** the measuring element is arranged as a flow obstacle onto which the body fluid can flow in a proximal flow cross-section of the collecting channel.

7. Process according to one of the claims 1 to 6, **characterized in that** the sensor member is connected to the lancing member by a clip, snap or latched connection.

8. Process according to one of the claims 1 to 7, **characterized in that** the sensor member is provided with a light guide in order to optically couple it to the measuring element.

9. Process according to one of the claims 1 to 8, **characterized in that** the measuring element is provided as a section of reagent tape by dividing a reagent tape provided with test chemistry into pieces.

10. Process according to one of the claims 1 to 9, **characterized in that** the measuring element is applied to an end face of the sensor member pointing towards the collecting channel in the connected state.

11. Process according to one of the claims 1 to 7, **characterized in that** the measuring element is formed by an end section of at least one electrode wire that is preferably continuously coated with a test reagent.

12. Process according to one of the claims 1 to 7, **characterized in that** the sensor member is formed by an electrochemical test strip, said test strip being provided with electrical conductor paths and at least one reagent field contacted by the conductor paths as a measuring element.

13. Process according to one of the claims 1 to 12, **characterized in that** the sensor member is provided with a holder that carries the measuring element and can be connected to the lancing member.

14. Process according to claim 13, **characterized in that** at least two parts of the holder are connected together while enclosing optical or electrical signal conductors, the holder parts being optionally provided with recesses for the defined positioning of the signal conductors.

15. Diagnostic test element (10) as a single-use article for analyzing a body fluid comprising a lancing member (14) that can puncture a body part, a preferably slot-shaped collecting channel (16) formed thereon for body fluid obtained by the puncture as well as a sensor member (18) designed to optically or electrochemically detect an analyte in the body fluid, **characterized in that** the sensor member (18) and the lancing member (14) are joined together as interlocking connecting components whereby a measuring element (42) of the sensor member engages into the collecting channel (16) via an insertion opening (46) in the lancing member (14).

16. Test element according to claim 15, **characterized in that** the volume of the collecting channel for taking up body fluid is less than 1 µl, preferably less than 100 nl.

## Patentansprüche

1. Verfahren zur Herstellung eines diagnostischen Testelements (10) zur Untersuchung einer Körperflüssigkeit bei welchem ein in ein Körperteil einstechbares Stechteil (14) mit einem vorzugsweise kapillaraktiven Sammelkanal (16) für durch den Einstich erhaltene Körperflüssigkeit versehen wird und ein Sensorteil (18) für eine optische oder elektrochemische Messung mit dem Stechteil verbunden wird, dass das Sensorteil (18) und das Stechteil (14) als ineinandergreifende Verbindungspartner zusammengefügt werden können, wobei ein Messelement (42) des Sensorteils über eine Einführöffnung (46) des Stechteils in den Sammelkanal eingeführt wird.

2. Verfahren nach Anspruch 1, **dadurch gekennzeichnet, dass** das Sensorteil und das Stechteil entlang einer in Längsrichtung des Sammelkanals verlaufenden Verbindungsachse zusammengefügt werden können.

3. Verfahren nach Anspruch 1 oder 2, **dadurch gekennzeichnet, dass** der Sammelkanal als ein an einer proximalen Mündung offener, vorzugsweise quer durchgehender Schlitz in dem Stechteil ausgebildet wird, und dass das Sensorteil über die proximale Mündung in den Schlitz eingeführt wird.

4. Verfahren nach Anspruch 1, **dadurch gekennzeichnet, dass** das Sensorteil quer in den längsseitig offenen Sammelkanal eingeführt wird.

5. Verfahren nach einem der Ansprüche 1 bis 4, **dadurch gekennzeichnet, dass** das Messelement im Inneren des Sammelkanals von der Kanalwandung galvanisch getrennt angeordnet wird.

6. Verfahren nach einem der Ansprüche 1 bis 5, **dadurch gekennzeichnet, dass** das Messelement als von der Körperflüssigkeit anströmbares Strömungshindernis in einem proximalen Fließquerschnitt des Sammelkanals angeordnet wird.

7. Verfahren nach einem der Ansprüche 1 bis 6, **dadurch gekennzeichnet**, das Sensorteil über eine Clip-, Schnapp- oder Rastverbindung mit dem Stechteil verbunden wird.

8. Verfahren nach einem der Ansprüche 1 bis 7, **dadurch gekennzeichnet, dass** das Sensorteil mit einem Lichtleiter zu seiner optischen Ankopplung an das Messelement versehen wird.

9. Verfahren nach einem der Ansprüche 1 bis 8, **dadurch gekennzeichnet, dass** das Messelement als Reagenzbandabschnitt durch Zerteilen eines mit einer Testchemie versehenen Reagenzbands bereitgestellt wird.

10. Verfahren nach einem der Ansprüche 1 bis 9, **dadurch gekennzeichnet, dass** das Messelement auf eine im Verbindungszustand in Richtung des Sammelkanals weisende Stirnfläche des Sensorteils aufgebracht wird.

11. Verfahren nach einem der Ansprüche 1 bis 7, **dadurch gekennzeichnet, dass** das Messelement durch einen Endabschnitt mindestens eines vorzugsweise kontinuierlich mit einem Testreagenz beschichteten Elektrodendrahts gebildet wird.

12. Verfahren nach einem der Ansprüche 1 bis 7, **dadurch gekennzeichnet, dass** das Sensorteil durch einen elektrochemisch arbeitenden Teststreifen gebildet wird, wobei der Teststreifen mit elektrischen Leiterbahnen und mindestens einem durch die Leiterbahnen kontaktierten Reagenzfeld als Messelement versehen wird.

13. Verfahren nach einem der Ansprüche 1 bis 12, **dadurch gekennzeichnet, dass** das Sensorteil mit einem das Messelement tragenden und mit dem Stechteil verbindbaren Halter versehen wird.

14. Verfahren nach Anspruch 13, **dadurch gekennzeichnet, dass** mindestens zwei Teile des Halters unter Einschluss von optischen oder elektrischen Signalleitern miteinander verbunden werden, wobei die Halterteile gegebenenfalls mit Ausnehmungen zur definierten Positionierung der Signalleiter versehen werden.

15. Diagnostisches Testelement (10) als Einmalartikel zur Untersuchung einer Körperflüssigkeit mit einem in ein Körperteil einstechbaren Stechteil (14), einem daran ausgebildeten, vorzugsweise schlitzförmigen Sammelkanal (16) für durch den Einstich erhaltene Körperflüssigkeit sowie einem zum optischen oder elektrochemischen Nachweis eines Analyten in der Körperflüssigkeit ausgebildeten Sensorteil (18), **dadurch gekennzeichnet, dass** das Sensorteil (18) und das Stechteil (16) als ineinandergreifende Verbindungspartner zusammengefügt sind, wobei ein Messelement (42) des Sensorteils über eine Einführöffnung (46) des Stechteils in den Sammelkanal (16) eingreift.

16. Diagnostisches Testelement nach Anspruch 15, **dadurch gekennzeichnet, dass** das Volumen des Sammelkanals zur Aufnahme von Körperflüssigkeit weniger als 1 µl, vorzugsweise weniger als 100 nl beträgt.

## Revendications

1. Procédé de production d'un élément de test diagnostique (10) pour analyser un fluide corporel dans lequel un élément de piqûre (14) qui peut piquer une partie du corps est prévu avec un canal de collecte préférablement capillaire (16) pour le fluide corporel obtenu par la piqûre et un élément de capteur (18) pour une mesure optique ou électrochimique est connecté à l'élément de piqûre, **caractérisé en ce que** l'élément de capteur (18) et l'élément de piqûre (14) peuvent être joints ensemble comme des composants à connexion par interverrouillage, dans lequel un élément de mesure (42) de l'élément de capteur est inséré à l'intérieur du canal de collecte à travers une ouverture d'insertion (46) de l'élément de piqûre.

2. Procédé selon la revendication 1, **caractérisé en ce que** l'élément de capteur et l'élément de piqûre peuvent être joints ensemble le long d'un axe de connexion s'étendant dans le sens longitudinal du canal de collecte.

3. Procédé selon la revendication 1 ou 2, **caractérisé en ce que** le canal de collecte est formé comme une fente continue préférablement transversale dans l'élément de piqûre, ladite fente étant ouverte au niveau d'une ouverture proximale, et **en ce que** l'élément de capteur est inséré à l'intérieur de la fente à travers l'ouverture proximale.

4. Procédé selon la revendication 1, **caractérisé en ce que** l'élément de capteur est inséré transversalement à l'intérieur du canal de collecte qui a un côté longitudinal ouvert.

5. Procédé selon l'une quelconque des revendications 1 à 4, **caractérisé en ce que** l'élément de mesure dans l'intérieur du canal de collecte est agencé de telle façon qu'il est galvaniquement séparé de la paroi du canal.

6. Procédé selon l'une quelconque des revendications 1 à 5, **caractérisé en ce que** l'élément de mesure est agencé comme un obstacle à l'écoulement sur lequel le fluide corporel peut s'écouler dans une section transversale proximale d'écoulement du canal de collecte.

7. Procédé selon l'une quelconque des revendications 1 à 6, **caractérisé en ce que** l'élément de capteur est connecté à l'élément de piqûre par une connexion à clip, à ergot ou à loquet.

8. Procédé selon l'une quelconque des revendications 1 à 7, **caractérisé en ce que** l'élément de capteur est prévu avec un guide de lumière afin de le coupler optiquement à l'élément de mesure.

9. Procédé selon l'une quelconque des revendications 1 à 8, **caractérisé en ce que** l'élément de mesure est fourni sous forme d'une section de bandelette réactive en divisant en morceaux une bandelette réactive dotée d'une chimie de test.

10. Procédé selon l'une quelconque des revendications 1 à 9, **caractérisé en ce que** l'élément de mesure est appliqué sur une face d'extrémité de l'élément de capteur pointant vers le canal de collecte à l'état connecté.

11. Procédé selon l'une quelconque des revendications 1 à 7, **caractérisé en ce que** l'élément de mesure est formé par une section d'extrémité d'au moins un fil électrode qui est préférablement revêtu de façon continue avec un réactif de test.

12. Procédé selon l'une quelconque des revendications 1 à 7, **caractérisé en ce que** l'élément de capteur est formé par une bande de test électrochimique, ladite bande de test étant prévue avec des chemins conducteurs électriques et au moins un champ de réactif au contact des chemins conducteurs comme un élément de mesure.

13. Procédé selon l'une quelconque des revendications 1 à 12, **caractérisé en ce que** l'élément de capteur est prévu avec un support qui supporte l'élément de mesure et peut être connecté à l'élément de piqûre.

14. Procédé selon la revendication 13, **caractérisé en ce qu'**au moins deux parties du support sont connectées ensemble tout en renfermant des conducteurs de signaux optiques ou électriques, les parties de support étant facultativement prévues avec des renfoncements pour le positionnement défini des conducteurs de signaux.

15. Élément de test diagnostique (10) comme un article à usage unique pour analyser un fluide corporel comprenant un élément de piqûre (14) qui peut piquer une partie du corps, un canal de collecte (16) préférablement en forme de fente formé sur celui-ci pour le fluide corporel obtenu par la piqûre, ainsi qu'un élément de capteur (18) conçu pour détecter optiquement ou électrochimiquement un analyte dans le fluide corporel, **caractérisé en ce que** l'élément de capteur (18) et l'élément de piqûre (14) sont joints ensemble comme des composants à connexion par interverrouillage, d'où il résulte qu'un élément de mesure (42) de l'élément de capteur s'engage à l'intérieur du canal de collecte (16) par une ouverture d'insertion (46) dans l'élément de piqûre (14).

16. Élément de test selon la revendication 15, **caractérisé en ce que** le volume du canal de collecte pour prélever un fluide corporel est inférieur à 1 µl, préférablement inférieur à 100 nl.
